# EUROPÄISCHE PATENTANMELDUNG

(11) **EP 1 559 783 A1**
(43) Veröffentlichungstag der Anmeldung: **03.08.2005**
(21) Anmeldenummer: 04001864.0
(22) Anmeldetag: 29.01.2004
(51) Int. Cl.: C12N 15/10

(54) **Verfahren zur chromatographischen Trennung eines Nukleinsäuregemisches**

(71) Anmelder: Qiagen GmbH, 40724 Hilden (DE)
(72) Erfinder: Müller, Markus, 41542 Dormagen (DE); Breitkopf, Lothar, 53115 Bonn (DE); Hucklenbroich, Jörg, 42085 Remscheid (DE)

(57) **Zusammenfassung**

Gegenstand der vorliegenden Erfindung ist ein Verfahren zur chromatographischen Trennung eines Nukleinsäuregemisches, insbesondere zur Trennung und Aufreinigung von Plasmid-DNA von anderen Bestandteilen des Nukleinsäuregemisches, insbesondere anderen Nukleinsäuren. Das erfindungsgemäße Verfahren zeichnet sich im besonderen dadurch aus, dass Plasmid-DNA ohne Zugabe von Ribonukleasen von kontaminierender RNA getrennt werden kann sowie durch die Verwendung kostengünstiger und umweltschonender Komponenten. Diese Parameter erlauben es, dieses Verfahren auch zur Produktion von Plasmid-DNA im Produktionsmaßstab ('large scale') einzusetzen. Weiterhin umfasst die vorliegende Erfindung die Verwendung der mittels des erfindungsgemäßen Verfahrens gewonnen Plasmid-DNA zur Herstellung eines Plasmid-DNA-haltigen Mittels zum Einsatz in der Gentherapie und genetischen Vakzinierung.

## Beschreibung

Gegenstand der vorliegenden Erfindung ist ein Verfahren zur chromatographischen Trennung eines Nukleinsäuregemisches, insbesondere zur Trennung und Aufreinigung von Plasmid-DNA von anderen Bestandteilen des Nukleinsäuregemisches, insbesondere anderen Nukleinsäuren. Das erfindungsgemäße Verfahren zeichnet sich im besonderen dadurch aus, dass Plasmid-DNA ohne Zugabe von Ribonukleasen von kontaminierender RNA getrennt werden kann sowie durch die Verwendung kostengünstiger und umweltschonender Komponenten. Diese Parameter erlauben es, dieses Verfahren auch zur Produktion von Plasmid-DNA im Produktionsmaßstab ('large scale') einzusetzen. Weiterhin umfasst die vorliegende Erfindung die Verwendung der mittels des erfindungsgemäßen Verfahrens gewonnen Plasmid-DNA zur Herstellung eines Plasmid-DNA-haltigen Mittels zum Einsatz in der Gentherapie und genetischen Vakzinierung.

Ein grundlegendes Problem der Aufreinigung von Plasmiden ist die Entfernung anderer Nukleinsäurespezies aus dem Produkt. Dieses Problem stellt sich vor allem in Bereichen, in denen eine besonders reine Plasmid-DNA-Präparation erforderlich ist, z.B. bei einem Einsatz der Plasmid-DNA in der Gentherapie. Die erwähnten anderen Nukleinsäurespezies sind vor allem die verschiedenen RNAs, aber auch genomische DNA und ssDNA (single stranded), etc. Eine besondere Schwierigkeit stellt die Entfernung der RNA dar. Aus dem Stand der Technik ist die Entfernung der RNA mit Hilfe von Ribonukleasen bekannt. Die RNA wird mittels der Ribonukleasen zu Ribonukleotiden abgebaut, welche in einem nachfolgenden chromatographischen Trennverfahren wesentlich leichter von der Plasmid-DNA getrennt werden können. Der massive Nachteil dieser Methode ist die Verwendung einer RNase, welche üblicherweise ein Fremdprotein darstellt. Die RNase wird aus tierischem Material, üblicherweise aus Rindern, gewonnen. Besonders bei der Herstellung von parenteralen Therapeutika zur Applikation am Menschen ist die Zugabe tierischer Proteine in Produktionsprozesse aufgrund einer möglichen Kontamination des Produktes mit bakteriellen, viralen oder proteinogenen Pathogenen auszuschließen. Dies gilt aufgrund der BSE-Problematik besonders für bovine Proteine.

Darüber hinaus stellt die Verwendung von RNase und auch von alkoholhaltigen Puffern einen großen Kostenfaktor dar. Gerade in der Produktion von Plasmid-DNA im großen Maßstab (large scale), also in Bereichen von etwa >2 g, ist dies ein nicht zu unterschätzender Kostenfaktor. Bei Verwendung von Alkoholen kommt zusätzlich eine Belastung der involvierten Mitarbeiter und der Umwelt als bedeutender faktor hinzu.

Ein generelles Problem bei der Aufreinigung von Nukleinsäuren aus prokaryontischen, aber auch aus eukaryontischen Zellen besteht in der zunächst durchzuführenden Lyse der Zellen, um eine Freisetzung der Nukleinsäuren zu bewirken. In dem erfindungsgemäßen Verfahren wird die von Birnborn und Dohly (Nucl. Acids Res. 7, pp. 1513 - 1522; 1979) grundsätzlich beschriebene alkalische Lyse bevorzugt, jedoch nicht darauf beschränkt. Weitere Möglichkeiten sind die Lyse durch Hitze oder Lyse in Anwesenheit von Detergenzien. Als ungeeignet hat sich die Lyse durch hohen Druck (French Press) erwiesen, das die entstehenden hohen Scheerkräfte sehr kleine Fragmente genomischer DNA entstehen, die praktisch nicht mehr von der Plasmid-DNA zu trennen sind.

Zur Aufreinigung der Nukleinsäuren aus einem solchen Lysat sind chromatographische Verfahren aus dem Stand der Technik bekannt. Hierbei sind generell zwei Verfahren zu unterscheiden. Zum einen ist die Methode nach Gillespie und Vogelstein (Proc. Natl. Acad. Sci., USA, 76 pp. 615 - 619; 1979) aus dem Stand der Technik bekannt. Bei dieser Methode erfolgt eine Aufreinigung der Nukleinsäure durch Bindung an Silicagel oder Diatomeenerde in Anwesenheit von chaotropen Salzen, wie z.B. GuHCl, Nal, etc. Im Gegensatz zum Anionenaustauscher erfolgt die Bindung der DNA in Anwesenheit von hohen Salzkonzentrationen, wogegen die Elution bei niedrigen Salzkonzentrationen erfolgt. Da bei dieser Methode die Bindung der Nukleinsäuren nach dem 'alles oder nichts'-Prinzip abläuft, ist keine quantitative Separation von RNA, ssDNA und Proteinen möglich. Daher sind die mittels dieser Methode gewonnenen DNA-Proben aufgrund ihrer Kontaminationen mit RNA und Proteinen nicht für einen Einsatz in der Gentherapie geeignet.

Zum zweiten ist die Reinigung mittels eines Anionenaustauschers zu nennen, wie sie in EP 0268 946 beschrieben ist. Hierbei werden Zellen, wie z.B. Bakterien, vorzugsweise mittels alkalischer Lyse aufgeschlossen. Die zellulären Proteine und genomische DNA werden mit Hilfe von Detergenzien und anschließender Zentrifugation abgetrennt. So gewonnene, Plasmid-DNA enthaltende Überstände werden als geklärtes Lysat (cleared lysate) bezeichnet. Das geklärte Lysat wird über eine Anionen-Austauscher-Säule (z.B. QIAGEN®, QIAGEN GmbH, Hilden, Deutschland) aufgereinigt, wobei eine quantitative Abtrennung von RNA und ssDNA erfolgt.

Das dem erfindungsgemäßen Verfahren zu Grunde liegende technische Problem ist die Aufreinigung von Plasmid-DNA aus einem Nukleinsäuregemisch und die Verbesserung der Trennung von Kontaminanten wie RNA, ssDNA und genomischer DNA ohne Verwendung einer RNase. Eine weitere der Erfindung zu Grunde liegende Aufgabe ist es, ein Verfahren bereitzustellen, dass eine Aufreinigung von Plasmiden auch im 'large scale' kostengünstig und umweltschonend erlaubt.

Überraschenderweise wird das der Erfindung zu Grunde liegende technische Problem durch ein Verfahren gemäß der Ansprüche gelöst. Bei dem erfindungsgemäßen Verfahren wird zur Trennung der Plasmid-DNA von den übrigen Bestandteilen des Nukleinsäuregemisches, insbesondere anderen Nukleinsäurespezies,
a) gegebenenfalls das Nukleinsäuregemisch mit einem oder mehreren Alkalisalzen und/oder Erdalkalisalzen in einer wässrigen Lösung auf eine Leitfähigkeit eingestellt wird, die einer Leitfähigkeit von 70 mS bis 95 mS bei einem pH von 4,8 bis 5,4 und bei einer Temperatur von 20°C entspricht, und
b) das Nukleinsäuregemisch mit einem chromatographischen Trägermaterial in Kontakt gebracht wird, und

a) das Trägermaterial anschließend mindestens einmal mit einer Lösung enthaltend ein Alkalisalz in einem Konzentrationsbereich von 900 mM bis 1800 mM bezogen auf einen pH von 7 bis 7,4 und/oder ein Erdalkalisalz in einem Konzentrationsbereich von 100 mM bis 240 mM bezogen auf einen pH von 7 bis 7,4 gewaschen wird, und
c) die an das chromatographischen Trägermaterial gebundene Plasmid DNA anschließend mit einer Lösung enthaltend ein Alkalisalz in einer Konzentration von 1300 mM oder höher bezogen auf einen pH von 7 bis 7,4 und/oder ein Erdalkalisalz in einer Konzentration von 270 mM oder höher bezogen auf einen pH von 7 bis 7,4 eluiert wird.

Zur Gewinnung eines Nukleinsäuregemisches müssen die Zellen, wobei es prokaryontische oder eukaryontische Zellen sein können, zunächst lysiert werden. Dies kann in der oben beschriebenen Weise geschehen. In dem erfindungsgemäßen Verfahren wird die von Birnborn und Dohly (Nucl. Acids Res. 7, pp. 1513 - 1522; 1979) grundsätzlich beschriebene alkalische Lyse bevorzugt, jedoch nicht darauf beschränkt. Weitere Möglichkeiten sind die Lyse durch Hitze oder Lyse in Anwesenheit von Detergenzien. Als ungeeignet hat sich die Lyse durch hohen Druck (French Press) erwiesen, da durch die entstehenden hohen Scheerkräfte sehr kleine Fragmente genomischer DNA entstehen, die praktisch nicht mehr von der Plasmid-DNA zu trennen sind.

Ein Nukleinsäuregemisch im Sinne der Erfindung kann dabei ein Zelllysat ebenso wie ein vorgereinigtes oder geklärtes Lysat sein, kann aber auch ein artifizielles Gemisch darstellen, bei dem Plasmid-DNA mit mindestens einer weiteren Nukleinsäurespezies und gegebenenfalls anderen Kontaminanten verunreinigt ist. In einer bevorzugten Ausführungsform des erfindungsgemäßen Verfahrens handelt es sich bei dem Nukleinsäuregemisch um ein prokaryontisches geklärtes Lysat.

Das erfindungsgemäße Verfahren gewährleistet die chromatographische Abtrennung der erwähnten Kontaminanten und liefert eine Plasmid-DNA, die die Anforderungen an die Reinheit zum Einsatz in der Gentherapie oder genetischen Vakzinierung erfüllt. Chromatographie versteht der Fachmann dabei als Sammelbegriff für die physikalisch-chemische Trennung von Substanzgemischen aufgrund ihrer unterschiedlichen Verteilung zwischen einer stationären Phase und einer mobilen Phase. In dem hier gegenständliche Verfahren wird zur Trennung der Plasmid-DNA von den Kontaminanten ein Anionenaustauscher-Material verwendet. Überraschenderweise zeigt insbesondere das im Handel erhältliche Material QIAGEN® (QIAGEN GmbH, Hilden, Deutschland) seine Eignung im erfindungsgemäßen Verfahren eingesetzt zu werden. Dieses Material ermöglicht mittels des erfindungsgemäßen Verfahrens eine sehr effiziente Abtrennung der RNA, aber auch von z.B. ssDNA, von Plasmid-DNA. RNA und ssDNA eluieren bei hier näher definierten Bedingungen in einem distinkten Peak, der in dem erfindungsgemäßen Verfahren sehr weit von dem ebenfalls sehr distinkten Peak der Plasmid-DNA entfernt liegt. Die Gefahr einer Coelution von Plasmid-DNA und RNA bzw. ssDNA ist somit im Vergleich zu den aus dem Stand der Technik bekannten Verfahren deutlich verringert.

Das unter der Bezeichnung QIAGEN® (QIAGEN GmbH, Hilden, Deutschland) erhältliche chromatographische Trägermaterial ist ein modifiziertes poröses anorganisches Material. Für ein chromatographische Trägermaterial im erfindungsgemäßen Verfahren kommen als anorganische Trägermaterialien Silicagel, Diatomeenerde, Glas, Aluminiumoxide, Titanoxide, Zirkonoxide, Hydroxylapatit und als organische Trägermaterialien solche wie Dextran, Agarose, Acrylamid, Polystyrolharze oder Copolymere aus den monomeren Bestandteilen der genannten Materialien in Frage.

Der Anionenaustauscher, der in dem erfindungsgemäßen Verfahren vorzugsweise eingesetzt wird, ist beispielsweise durch die Umsetzung eines der oben genannten Trägermaterialien in einem ersten Schritt mit einem Silanisierungsreagenz der allgemeinen Formel I

R¹R²R³SiR⁴ (I)

wobei
R¹ ein Alkoxyrest mit 1 bis 10 C-Atomen, insbesondere -OCH₃, -OC₂H₅ oder - OC₃H₇, oder ein Halogenatom, insbesondere -Cl, oder eine Dialkylaminogruppe mit identischen oder unterschiedlichen Alkylresten mit 1 bis 6 C-Atomen;
R² und R³ unabhängig voneinander ein Kohlenwasserstoffrest mit 1 bis 10 C-Atomen, insbesondere -CH₃, -C₂H₅ oder -C₃H₇, oder ein Alkoxyrest mit 1 bis 10 C-Atomen, insbesondere -OCH₃, -OC₂H₅ oder -OC₃H₇, oder ein Halogenatom oder ein durch mindestens ein Sauerstoffatom oder eine Aminogruppe unterbrochener Alkylrest mit 4 bis 20 C-Atomen, wobei dieser Rest auch ein ein- oder mehrfach durch Halogen, Cyano, Nitro, Amino, Monoalkylamino, Dialkylamino, Hydroxy oder Aryl substituiert sein kann;
R⁴ eine Kohlenwasserstoffkette mit 1 bis 20 C-Atomen oder ein durch mindestens ein Sauerstoffatom oder eine Aminogruppe unterbrochener Alkylrest, wobei dieser Rest auch ein- oder mehrfach mit Halogen, Cyano, Nitro, Amino, Monoalkylamino, Dialkylamino, Alkoxy, Hydroxy, Aryl und/oder Epoxy substituiert sein kann, insbesondere 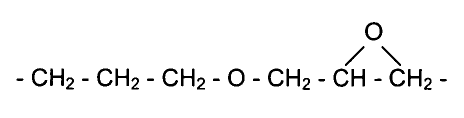 ist,
gefolgt von einem zweiten Schritt, wobei der im ersten Schritt modifizierte Träger umgesetzt wird mit einem Reagenz der allgemeinen Formel II

X-R-Y (II)

wobei
X eine Amino-, Hydroxy-, Epoxy-Gruppe oder ein Halogenatom,
R eine Kohlenwasserstoffkette mit 2 bis 20 C-Atomen oder ein durch mindestens ein Sauerstoffatom oder eine Aminogruppe unterbrochener Alkylrest, wobei dieser Rest auch ein- oder mehrfach mit Halogen, Cyano, Nitro, Amino, Monoalkylamino, Dialkylamino, Alkoxy, Hydroxy, Aryl und/oder Epoxy substituiert sein kann,
Y ein Kohlenwasserstoffrest mit Anionenaustauscher bildenden funktionellen Gruppen mit 1 bis 10 C-Atomen, der ein- oder mehrfach mit Amino-, Monoalkylamino-, Dialkylamino-, Trialkylammonium substituiert sein kann, ist, wie auch in EP 0 743 949, Seite 4 bis 5, auf die hier inhaltlich Bezug genommen wird, beschrieben.

In einer bevorzugten Ausführung wird das Nukleinsäuregemisch gemäß des fakultativen Schritts a) des oben beschriebenen erfindungsgemäßen Verfahrens mit einem oder mehreren Alkalisalzen und/oder Erdalkalisalzen in einer wässrigen Lösung auf eine Leitfähigkeit eingestellt, die einer Leitfähigkeit von 70 mS bis 95 mS bei einem pH von 4,8 bis 5,4 und bei einer Temperatur von 20°C entspricht. Dem Fachmann ist bekannt, dass die Leitfähigkeit einer salzhaltigen Lösung in Abhängigkeit von der jeweiligen Temperatur und dem pH-Wert deutlich variieren kann und er kann aufgrund der ihm geläufigen Gesetzmäßigkeiten eine entsprechende Anpassung der Leitfähigkeiten bei Veränderungen der Temperatur und/oder des pH-Wertes vornehmen, sodass eine Durchführung des erfindungsgemäßen Verfahrens auch bei Veränderung dieser Parameter problemlos möglich ist.

Die in dem erfindungsgemäßen Verfahren vorzugsweise eingesetzten Salze sind Alkalisalze, also Salze, bei denen die kationische Komponente bzw. ein Teil der kationischen Komponente aus einem Element der ersten Hauptgruppe des Periodensystems der Elemente stammt, und/oder sind Erdalkalisalze, also Salze, bei denen die kationische Komponente bzw. ein Teil der kationischen Komponente aus einem Element der zweiten Hauptgruppe des Periodensystems der Elemente stammt. Besonders bevorzugt handelt es sich bei den Alkalisalzen um Alkalihalogenide und bei den Erdalkalisalzen um Erdalkalihalogenide. Besonders bevorzugt ist die Verwendung der Alkalihalogenide KCl, NaCl, CsCl und/oder LiCl sowie des Erdalkalihalogenids CaCl₂. Alternativ zu den Alkali- bzw. Erdalkalisalzen kann auch ein Ammoniumsalz (Pseudoalkalisalz), bevorzugt ein Ammoniumsalz einer Carbonsäure, besonders bevorzugt Ammoniumacetat in dem erfindungsgemäßen Verfahren verwendet werden. Ganz besonders bevorzugt handelt es sich bei den verwendeten Salzen um KCl und/oder NaCl. Neben den einzelnen Salzen können in dem erfindungsgemäßen Verfahren auch Gemische aus verschiedenen Alkalisalzen und/oder Erdalkalisalzen verwendet werden.

In dem oben beschriebenen Waschschritt c) zur Elution der Kontaminanten werden Alkalisalze in einem Konzentrationsbereich von 900 mM bis 1800 mM bezogen auf einen pH von 7 bis 7,4 und/oder Erdalkalisalze in einem Konzentrationsbereich von 100 mM bis 240 mM bezogen auf einen pH von 7 bis 7,4 eingesetzt. Für den Waschschritt können grundsätzlich alle für den Fachmann sinnvoll erscheinenden wässrigen Lösungen eingesetzt werden, z.B. gepufferte Systeme wie beispielsweise, aber nicht beschränkt auf, Tris-, Kaliumacetat-, Borat- oder MOPS-gepufferte Systeme, oder alternativ ungepufferte Systeme, d.h. die Salze werden ausschließlich in Wasser gelöst. Aus unterschiedlichen Puffersystemen können potentiell auch unterschiedliche pH-Werte resultieren bzw. diese können eingestellt werden. Die hier gewählten Konzentrationsbereiche beziehen sich auf einen pH-Wert von 7 bis 7,4, grundsätzlich kann der pH-Wert der Waschlösung in dem oben erwähnten pH-Bereich aber variiert werden. Dem Fachmann ist bekannt, dass bei Veränderung des pH-Wertes einer solchen Waschlösung auch die Konzentration der darin enthaltenen Salze geändert werden muss, um den gleichen Effekt, in diesem Fall also die Elution der Kontaminanten, zu erzielen, d.h. es kommt bei Durchführung des Verfahrens zu einer Verschiebung der Elutionspunkte von Kontaminanten (z.B. RNA) und Plasmid-DNA, bei gleichem pH-Wert von Wasch- und Elutionslösung nicht aber zu einer Verschiebung des Verhältnisses der Elutionspunkte, was bedeutet, dass der Abstand der Elutionspeaks der unterschiedlichen Nukleinsäurespezies vorteilhafterweise immer gleich bleibt. Die hierzu erforderlichen Parameter kann der Fachmann anhand seines Fachwissens ohne erfinderisches Zutun vornehmen. Das erfindungsgemäße Verfahren umfasst mindestens einen Waschschritt, es können aber auch mehrere, in der Anzahl dem Fachmann sinnvoll erscheinend, Waschschritte, auch mit untereinander verschiedenen erfindungsgemäßen Waschpuffern, durchgeführt werden.

In einer bevorzugten Ausführungsform wird mindestens ein Waschschritt durchgeführt mit einer Lösung enthaltend KCl in einem Konzentrationsbereich von 1100 mM bis 1800 mM bezogen auf einen pH von 7 bis 7,4, besonders bevorzugt wird mindestens ein Waschschritt durchgeführt mit einer Lösung enthaltend KCl in einem Konzentrationsbereich von 1300 mM bis 1700 mM bezogen auf einen pH von 7 bis 7,4.

In einer weiteren bevorzugten Ausführungsform wird mindestens ein Waschschritt durchgeführt mit einer Lösung enthaltend NaCl in einem Konzentrationsbereich von 950 mM bis 1200 mM bezogen auf einen pH von 7 bis 7,4, besonders bevorzugt wird mindestens ein Waschschritt durchgeführt mit einer Lösung enthaltend NaCl in einem Konzentrationsbereich von 1100 mM bis 1150 mM bezogen auf einen pH von 7 bis 7,4.

In dem oben beschriebenen Elutionsschritt d) zur Elution der Plasmid-DNA werden Alkalisalze in einer Konzentration von 1300 mM oder höher bezogen auf einen pH von 7 bis 7,4 und/oder Erdalkalisalze in einer Konzentration von 270 mM oder höher bezogen auf einen pH von 7 bis 7,4 eingesetzt. Für den Elutionsschritt können analog zum Waschschritt grundsätzlich alle für den Fachmann sinnvoll erscheinenden wässrigen Lösungen eingesetzt werden, z.B. gepufferte Systeme wie beispielsweise, aber nicht beschränkt auf, Tris-, Kaliumacetat-, Borat- oder MOPS-gepufferte Systeme, oder alternativ ungepufferte Systeme, d.h. die Salze werden ausschließlich in Wasser gelöst. Aus unterschiedlichen Puffersystemen können potentiell auch unterschiedliche pH-Werte resultieren bzw. diese können eingestellt werden. Die hier gewählten Konzentrationsbereiche beziehen sich auf einen pH-Wert von 7 bis 7,4, grundsätzlich kann der pH-Wert der Elutionslösung in dem oben erwähnten pH-Bereich aber variiert werden. Dem Fachmann ist bekannt, dass bei Veränderung des pH-Wertes einer solchen Elutionslösung auch die Konzentration der darin enthaltenen Salze geändert werden muss, um den gleichen Effekt, in diesem Fall also die Elution der Plasmid-DNA, zu erzielen, d.h. es kommt bei Durchführung des Verfahrens zu einer Verschiebung der Elutionspunkte von Kontaminanten (z.B. RNA) und Plasmid-DNA, bei gleichem pH-Wert von Wasch- und Elutionslösung nicht aber zu einer Verschiebung des Verhältnisses der Elutionspunkte, was bedeutet, dass der Abstand der Elutionspeaks der unterschiedlichen Nukleinsäurespezies vorteilhafterweise immer gleich bleibt. Die hierzu erforderlichen Parameter kann der Fachmann anhand seines Fachwissens ohne erfinderisches Zutun vornehmen.

In einer bevorzugten Ausführungsform wird der Elutionsschritt durchgeführt mit einer Lösung enthaltend KCl in einer Konzentration von 1900 mM oder höher bezogen auf einen pH von 7 bis 7,4. Dabei ist die Konzentration von KCl nach oben nur durch seine Löslichkeit in der verwendeten Lösung begrenzt.

In einer weiteren bevorzugten Ausführungsform wird der Elutionsschritt durchgeführt mit einer Lösung enthaltend NaCl in einer Konzentration 1300 mM oder höher bezogen auf einen pH von 7 bis 7,4. Dabei ist die Konzentration von NaCl nach oben nur durch seine Löslichkeit in der verwendeten Lösung begrenzt.

Die Einstellung der Leitfähigkeit des Nukleinsäuregemisches vor in Kontakt bringen des Nukleinsäuregemisches mit dem chromatographischen Trägermaterial erfolgt, wie oben bereits erwähnt, ebenfalls mit Alkalisalzen und/oder Erdalkalisalzen. In einer besonders bevorzugten Ausführungsform wird das Nukleinsäuregemisch mit KCl auf eine Leitfähigkeit eingestellt, die einer Leitfähigkeit von 70 mS bis 85 mS bei einem pH von 4,8 bis 5,4 und bei einer Temperatur von 20°C entspricht, ganz besonders bevorzugt auf eine Leitfähigkeit, die einer Leitfähigkeit von 70 mS bis 80 mS bei einem pH von 4,8 bis 5,4 und bei einer Temperatur von 20°C entspricht. In einer weiteren besonders bevorzugten Ausführungsform wird das Nukleinsäuregemisch mit NaCl auf eine Leitfähigkeit eingestellt, die einer Leitfähigkeit von 70 mS bis 95 mS bei einem pH von 4,8 bis 5,4 und bei einer Temperatur von 20°C entspricht, ganz besonders bevorzugt auf eine Leitfähigkeit, die einer Leitfähigkeit von 85 mS bis 95 mS bei einem pH von 4,8 bis 5,4 und bei einer Temperatur von 20°C entspricht.

In einer bevorzugten Ausführungsform wird in dem erfindungsgemäßen Verfahren mindestens in dem oben mit c) gekennzeichneten Waschschritt des chromatographischen Trägermaterials das Alkalihalogenid KCl eingesetzt.

Das erfindungsgemäße Verfahren wird bevorzugt bei Raumtemperatur durchgeführt. Raumtemperatur bedeutet im vorliegenden Falle, dass das Verfahren bei normalen Prozessbedingungen durchgeführt wird, entsprechend etwa einem Rahmen von 18°C bis 25°C. Grundsätzlich kann das Verfahren bei allen dem Fachmann sinnvoll erscheinenden Temperaturen durchgeführt werden.

Vorzugsweise eignet sich das erfindungsgemäße Verfahren zur Aufreinigung von Plasmid-DNA. Überraschender- und vorteilhafterweise hat sich gezeigt, dass Plasmide unterschiedlichster Größe keine signifikanten Unterschiede in den Elutionspunkten, d.h. in den Salzkonzentrationen, bei welchen eine Elution der Plasmid-DNA vom chromatographischen Trägermaterial erfolgt, zeigen. Eine Adaption der Parameter des Verfahrens, wie beispielsweise Salzkonzentrationen oder pH-Werte, an unterschiedliche Plasmidgrößen ist somit nicht erforderlich.

Da mit dem Erfindungsgegenstand ein Verfahren zur Verfügung steht, in dem auch im 'large scale' Plasmid zur Herstellung eines Plasmid-DNA-haltigen Mittels zum Einsatz in der Gentherapie oder genetischen Vakzinierung gewonnen werden können, kann vorteilhafterweise eine Endotoxinentfernung völlig problemlos in das Verfahren eingebaut werden. Hierzu können nahezu alle aus dem Stand der Technik bekannten Verfahren benutzt werden. Beispielsweise kann das geklärte Lysat mit einem aus dem Stand der Technik bekannten Endotoxin-Entfernungspuffer (z.B. enthaltend Triton X 100, Triton X 114, Polymyxin, etc.) versetzt werden und ohne Änderung im vorliegenden erfindungsgemäßen Verfahrens weiter verwendet werden.

### Abbildungen

### Abbildung 1:

Aufgetragen ist die Extinktion bei 254 nm des Durchflusses einer mit QIAGEN®-Chromatographiematerial gefüllten HPLC-Säule gegen die KCl-Konzentration. Dargestellt ist die Elution unterschiedlicher Nukleinsäurespezies mit steigender KCI-Konzentration. Die Versuchsbedingungen sind in Beispiel 2 näher erläutert.

### Beispiele

### Beispiel 1: Aufreinigung von pCMVβ aus E.coli DH5α

Aus 30 L einer Übernacht-Fermentationskultur von E.coli DH5α, enthaltend pCMVβ-Plasmid, wurde durch Zentrifugation 1 kg Biomasse gewonnen. Die Biomasse wurde in 15 L eines Resuspendierungspuffers (10 mM EDTA; 50 mM Tris/HCl pH 8) resuspendiert und anschließend mit 15 L eines Lysepuffers (200 mM NaOH; 1 % (w/v) SDS) für 10 Minuten bei Raumtemperatur inkubiert. Im Anschluss wurden 15 L eines Neutralisationspuffers (3 M Kaliumacetat, pH 5,5) zugegeben. Das in diesem Schritt entstandene Präzipitat (Proteine, Membranbestandteile, genomische DNA, etc.) wurde anschließend entfernt. Das so vorgeklärte Lysat wurde anschließend noch filtriert, wodurch ein geklärtes Lysat erzeugt wurde. Das geklärte Lysat wies in der Folge einen pH von 5,2 auf und wurde bei einer Temperatur von 20°C mit 3 M KCl auf eine Leitfähigkeit von 80 mS eingestellt.

Eine Chromatographiesäule wurde mit QIAGEN®-Chromatographiematerial gefüllt (Säulenvolumen ca. 7 L) und mit 10 Säulenvolumina eines Äquilibrierungspuffers (20 mM Kaliumacetat) bei einer Flussrate von 3,3 cm/min äquilibriert. Das geklärte Lysat wurde nach der Äquilibrierung des Chromatographiematerials auf die Säule geladen, der Lauf erfolgte mit einer Flussrate von 1,1 cm/min. Anschließend wurden noch einmal 5 Säulevolumina Äquilibrierungspuffer (20 mM Kaliumacetat) mit einer Flussrate von 3,3 cm/min über die Säule gegeben.

Die Säule wurde direkt im Anschluss mit 10 Säulenvolumina einer KCl-Lösung (1350mM KCl; 50 mM Tris/HCl, pH 7,2) bei einer Flussrate von 3,3 cm/min gewaschen. Darauf folgend wurden die Plasmide mit einem Säulenvolumen eines Elutionspuffers (1600 mM NaCl; 50 mM Tris/HCl, pH 7,2) eluiert. Nach anschließender Ultra-/Diafiltration und finaler Sterilfiltration ergab sich eine Ausbeute von ca. 400 mg pCMVβ.

### Beispiel 2:

In 4 unterschiedlichen Ansätzen wurden je 600 µg eines aufgereinigten Plasmids (1: Plasmid A [3266 bp]; 2: Plasmid B [7200 bp];3: Plasmid C [7687 bp]; 4: Plasmid D [19535 bp]) zusammen mit jeweils 600 µg aufgereinigter RNA (E.coli HB101) in 5 ml 60 mM Kaliumacetat gelöst.

Eine HPLC-Säule (Säulenvolumen 4,4 ml) wurde mit QIAGEN®-Chromatographiematerial gefüllt und mit 2 Säulenvolumina (Flussrate 1 ml/min) Äquilibrierungspuffer (60 mM Kaliumacetat) äquilibriert. Anschließend wurden in 4 einzelnen Säulen mit jeweils frisch gepackten HPLC-Säulen die 5 ml des Plasmid-RNA-Gemisches mit 1 ml/min durch die Säule gegeben, die Säule anschließend mit 3 Säulenvolumina 60 mM Kaliumacetat gespült.

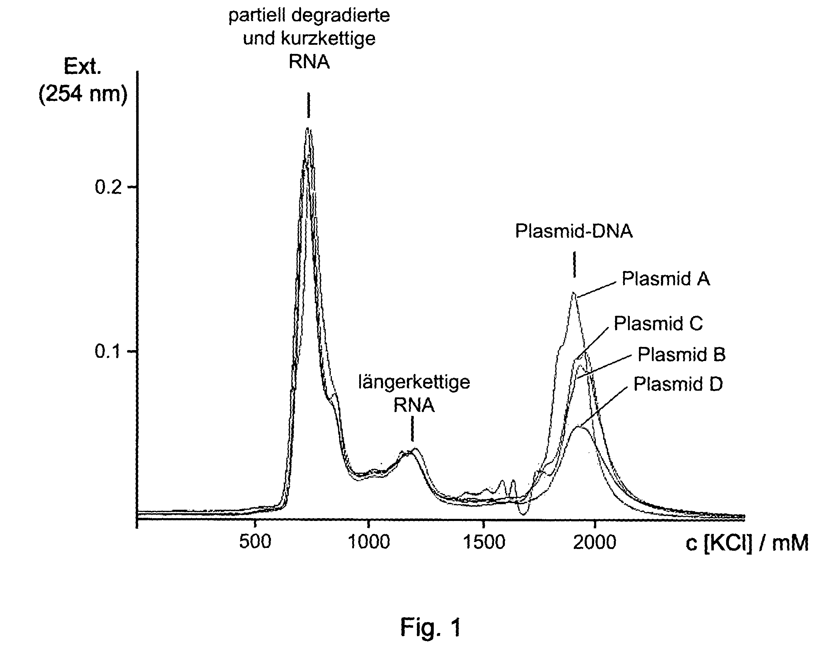

In einem kontinuierlichen Gradienten wurde ein Tris-Puffer über die Säule gegeben (50 mM Tris/HCl; pH 7,2; Gradient 0 bis 3 M KCl) und die Elution von DNA und RNA mittels eines Photometers (Extinktionsmessung bei 254 nm) bestimmt. Dabei konnte gezeigt werden, dass bereits teilweise degradierte und kurzkettige RNA in einem distinkten Peak (Maximum bei 780 mM KCl) eluiert wird, gefolgt von einem nur leicht diffusen Peak längerkettiger RNA (Maximum bei 1120 mM KCl, Ende der Elution bei 1310 mM KCl). Die Elution der Plasmid-DNA erreicht ein Maximum bei 1900 mM KCl und endet bei 2150 mM KCl. Die Ergebnisse sind als übereinandergelegte Spuren in Abbildung 1 dargestellt. Es wird deutlich, dass die Plasmide vorteilhafterweise unabhängig von ihrer Größe eluieren.

## Patentansprüche

1. Verfahren zur chromatographischen Trennung eines Nukleinsäuregemisches, wobei Plasmid-DNA von anderen Bestandteilen des Gemisches, insbesondere anderen Nukleinsäuren, getrennt wird, **dadurch gekennzeichnet, dass**
b) gegebenenfalls das Nukleinsäuregemisch mit einem oder mehreren Alkalisalzen und/oder Erdalkalisalzen in einer wässrigen Lösung auf eine Leitfähigkeit eingestellt wird, die einer Leitfähigkeit von 70 mS bis 95 mS bei einem pH von 4,8 bis 5,4 und bei einer Temperatur von 20°C entspricht, und
c) das Nukleinsäuregemisch mit einem chromatographischen Trägermaterial in Kontakt gebracht wird, und
d) das Trägermaterial anschließend mindestens einmal mit einer Lösung enthaltend ein Alkalisalz in einem Konzentrationsbereich von 900 mM bis 1800 mM bezogen auf einen pH von 7 bis 7,4 und/oder ein Erdalkalisalz in einem Konzentrationsbereich von 100 mM bis 240 mM bezogen auf einen pH von 7 bis 7,4 gewaschen wird, und
e) die an das chromatographischen Trägermaterial gebundene Plasmid DNA anschließend mit einer Lösung enthaltend ein Alkalisalz in einer Konzentration von 1300 mM oder höher bezogen auf einen pH von 7 bis 7,4 und/oder ein Erdalkalisalz in einer Konzentration von 270 mM oder höher bezogen auf einen pH von 7 bis 7,4 eluiert wird.

2. Verfahren gemäß Anspruch 1, **dadurch gekennzeichnet, dass** das Alkalisalz ein Alkalihalogenid ist und das Erdalkalisalz ein Erdalkalihalogenid ist.

3. Verfahren gemäß Anspruch 2, **dadurch gekennzeichnet, dass** das Alkalihalogenid NaCl, KCl, CsCl und/oder LiCl ist und das Erdalkalihalogenid CaCl₂ ist.

4. Verfahren gemäß der Ansprüche 1 bis 3, **dadurch gekennzeichnet, dass** das Nukleinsäuregemisch mit KCI auf eine Leitfähigkeit eingestellt wird, die einer Leitfähigkeit von 70 mS bis 85 mS bei einem pH von 4,8 bis 5,4 und bei einer Temperatur von 20°C entspricht.

5. Verfahren gemäß Anspruch 4, **dadurch gekennzeichnet, dass** das Nukleinsäuregemisch mit KCl auf eine Leitfähigkeit eingestellt wird, die einer Leitfähigkeit von 70 mS bis 80 mS bei einem pH von 4,8 bis 5,4 und bei einer Temperatur von 20°C entspricht.

6. Verfahren gemäß der Ansprüche 1 bis 3, **dadurch gekennzeichnet, dass** das Nukleinsäuregemisch mit NaCl auf eine Leitfähigkeit eingestellt wird, die einer Leitfähigkeit von 70 mS bis 95 mS bei einem pH von 4,8 bis 5,4 und bei einer Temperatur von 20°C entspricht.

7. Verfahren gemäß Anspruch 6, **dadurch gekennzeichnet, dass** das Nukleinsäuregemisch mit NaCl auf eine Leitfähigkeit eingestellt wird, die einer Leitfähigkeit von 85 mS bis 95 mS bei einem pH von 4,8 bis 5,4 und bei einer Temperatur von 20°C entspricht.

8. Verfahren gemäß der Ansprüche 1 bis 7, **dadurch gekennzeichnet, dass** der/die Waschschritt/e aus Schritt b) von Anspruch 1 mit einer Lösung enthaltend KCI in einem Konzentrationsbereich von 1100 mM bis 1800 mM bezogen auf einen pH von 7 bis 7,4 durchgeführt wird/werden.

9. Verfahren gemäß Anspruch 8, **dadurch gekennzeichnet, dass** der/die Waschschritt/e aus Schritt b) von Anspruch 1 mit einer Lösung enthaltend KCl in einem Konzentrationsbereich von 1300 mM bis 1700 mM bezogen auf einen pH von 7 bis 7,4 durchgeführt wird/werden.

10. Verfahren gemäß der Ansprüche 1 bis 7, **dadurch gekennzeichnet, dass** der/die Waschschritt/e aus Schritt b) von Anspruch 1 mit einer Lösung enthaltend NaCl in einem Konzentrationsbereich von 950 mM bis 1200 mM bezogen auf einen pH von 7 bis 7,4 durchgeführt wird/werden.

11. Verfahren gemäß Anspruch 10, **dadurch gekennzeichnet, dass** der/die Waschschritt/e aus Schritt b) von Anspruch 1 mit einer Lösung enthaltend NaCl in einem Konzentrationsbereich von 1100 mM bis 1150 mM bezogen auf einen pH von 7 bis 7,4 durchgeführt wird/werden.

12. Verfahren gemäß der Ansprüche 1 bis 11, **dadurch gekennzeichnet, dass** der Elutionsschritt aus Schritt c) von Anspruch 1 mit einer Lösung enthaltend KCl in einer Konzentration von 1900 mM oder höher bezogen auf einen pH von 7 bis 7,4 durchgeführt wird.

13. Verfahren gemäß der Ansprüche 1 bis 11, **dadurch gekennzeichnet, dass** der Elutionsschritt aus Schritt c) von Anspruch 1 mit einer Lösung enthaltend NaCl in einer Konzentration von 1300 mM oder höher bezogen auf einen pH von 7 bis 7,4 durchgeführt wird.

14. Verfahren gemäß der Ansprüche 1 bis 13, **dadurch gekennzeichnet, dass** das chromatographische Trägermaterial ein Anionenaustauscher ist.

15. Verfahren gemäß der Ansprüche 1 bis 14, **dadurch gekennzeichnet, dass** als chromatographische Trägermaterial Silicagel, Diatomeenerde, Glas, Aluminiumoxide, Titanoxide, Zirkonoxide, Hydroxylapatit, Dextran, Agarose, Acrylamid, Polystyrolharze oder Copolymere der genannten Materialien verwendet werden.

16. Verfahren gemäß Anspruch 15, **dadurch gekennzeichnet, dass** das chromatographische Trägermaterial erhältlich ist durch Umsetzung eines der in Anspruch 15 genannten Trägermaterialien in einem ersten Schritt mit einem Silanisierungsreagenz der allgemeinen Formel I
R¹R²R³SiR⁴ (I)
wobei
R¹ ein Alkoxyrest mit 1 bis 10 C-Atomen, insbesondere -OCH₃, -OC₂H₅ oder - OC₃H₇, oder ein Halogenatom, insbesondere -Cl, oder eine Dialkylaminogruppe mit identischen oder unterschiedlichen Alkylresten mit 1 bis 6 C-Atomen;
R² und R³ unabhängig voneinander ein Kohlenwasserstoffrest mit 1 bis 10 C-Atomen, insbesondere -CH₃, -C₂H₅ oder -C₃H₇, oder ein Alkoxyrest mit 1 bis 10 C-Atomen, insbesondere -OCH₃, -OC₂H₅ oder -OC₃H₇, oder ein Halogenatom oder ein durch mindestens ein Sauerstoffatom oder eine Aminogruppe unterbrochener Alkylrest mit 4 bis 20 C-Atomen, wobei dieser Rest auch ein ein- oder mehrfach durch Halogen, Cyano, Nitro, Amino, Monoalkylamino, Dialkylamino, Hydroxy oder Aryl substituiert sein kann;
R⁴ eine Kohlenwasserstoffkette mit 1 bis 20 C-Atomen oder ein durch mindestens ein Sauerstoffatom oder eine Aminogruppe unterbrochener Alkylrest, wobei dieser Rest auch ein- oder mehrfach mit Halogen, Cyano, Nitro, Amino, Monoalkylamino, Dialkylamino, Alkoxy, Hydroxy, Aryl und/oder Epoxy substituiert sein kann, insbesondere 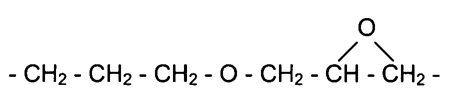 ist,
gefolgt von einem zweiten Schritt, wobei der im ersten Schritt modifizierte Träger umgesetzt wird mit einem Reagenz der allgemeinen Formel II
X-R-Y (II)
wobei
X eine Amino-, Hydroxy-, Epoxy-Gruppe oder ein Halogenatom,
R eine Kohlenwasserstoffkette mit 2 bis 20 C-Atomen oder ein durch mindestens ein Sauerstoffatom oder eine Aminogruppe unterbrochener Alkylrest, wobei dieser Rest auch ein- oder mehrfach mit Halogen, Cyano, Nitro, Amino, Monoalkylamino, Dialkylamino, Alkoxy, Hydroxy, Aryl und/oder Epoxy substituiert sein kann,
Y ein Kohlenwasserstoffrest mit Anionenaustauscher bildenden funktionellen Gruppen mit 1 bis 10 C-Atomen, der ein- oder mehrfach mit Amino-, Monoalkylamino-, Dialkylamino-, Trialkylammonium substituiert sein kann, ist.

17. Verfahren gemäß der Ansprüche 1 bis 16, **dadurch gekennzeichnet, dass** das Verfahren bei Raumtemperatur durchgeführt wird.

18. Verfahren gemäß der Ansprüche 1 bis 3, **dadurch gekennzeichnet, dass** mindestens in Schritt b) von Anspruch 1 als Salz KCl verwendet wird.

19. Verfahren gemäß Anspruch 1, **dadurch gekennzeichnet, dass** in den Schritten a), c) und d) des Anspruchs 1 auch Gemische verschiedener Alkalisalze und/oder Erdalkalisalze verwendet werden können.

20. Verfahren gemäß der Ansprüche 1 bis 19, **dadurch gekennzeichnet, dass** das Nukleinsäuregemisch ein geklärtes Lysat prokaryontischer Zellen ist.

21. Verwendung des Verfahrens gemäß der Ansprüche 1 bis 20 zur Aufreinigung von Plasmid-DNA.

22. Verwendung der mittels eines Verfahrens gemäß der Ansprüche 1 bis 20 gewonnen Plasmide zur Herstellung eines Plasmid-DNA-haltigen Mittels zum Einsatz in der Gentherapie oder genetischen Vakzinierung.
